# EUROPÄISCHE PATENTSCHRIFT

(11) **EP 3 930 786 B1**
(45) Veröffentlichungstag und Bekanntmachung des Hinweises auf die Patenterteilung: **11.11.2026**
(21) Anmeldenummer: 20707062.4
(22) Anmeldetag: 24.02.2020
(51) Int. Cl.: A61M 1/16, A61M 1/36

(54) **DIALYSATOR UND DIALYSEVORRICHTUNG**
DIALYSER AND DIALYSIS DEVICE
DIALYSEUR ET DISPOSITIF DE DIALYSE

(30) Priorität: 26.02.2019 DE 102019104837
(43) Veröffentlichungstag der Anmeldung: 05.01.2022
(73) Patentinhaber: B. Braun Avitum AG, 34212 Melsungen (DE)
(72) Erfinder: RITTER, Kai-Uwe, 34212 Melsungen (DE)
(74) Vertreter: Winter, Brandl - Partnerschaft mbB
(86) Internationale Anmeldenummer: PCT/EP2020/054736
(87) Internationale Veröffentlichungsnummer: WO 2020/173857

(56) Entgegenhaltungen:
- CN-U- 203 915 767
- DE-C2- 19 728 071
- US-A- 4 293 413
- US-A1- 2007 102 340
- US-A1- 2009 084 720
- US-A1- 2015 367 062
- US-B2- 7 938 967

## Beschreibung

Die vorliegende Erfindung bezieht sich auf einen Dialysator für eine extrakorporale Blutbehandlung mit einem langgestreckten Dialysatorgehäuse, zumindest einer Dialysemembran, welche einen Innenraum des Dialysatorgehäuses in eine mit zwei Dialysierflüssigkeitsanschlüssen versehene Dialysierflüssigkeitskammer und eine mit zwei Blutanschlüssen versehene Blutkammer trennt. Des Weiteren bezieht sich die Erfindung auf eine Dialysevorrichtung mit einem solchen Dialysator.

### Stand der Technik

Bekannte Dialysatoren weisen in der Regel vier Anschlüsse auf, mit denen sie einerseits mit einem Leitungssystem einer Dialysemaschine und anderseits mit einem mit einem Patienten verbundenen extrakorporalen Blutsystem verbindbar sind. Die genannten Anschlüsse teilen sich auf in zwei Anschlüsse zur Verbindung mit dem extrakorporalen Blutsystem bzw. Blutkreislauf, über die zu reinigendes Blut zum Dialysator hin und vom Dialysator abgeführt wird, im Folgenden auch als Blutzuleitungsanschluss und Blutableitungsanschluss bezeichnet, und zwei Anschlüsse zur Verbindung mit der Dialysemaschine, über die Dialysierflüssigkeit zum Dialysator hin und vom Dialysator abgeführt wird, im Folgenden auch als Dialysierflüssigkeitszuleitungsanschluss und Dialysierflüssigkeitsableitungsanschluss bezeichnet.

Vor dem Beginn eines Blutbehandlungsverfahrens ist es notwendig, den extrakorporalen Blutkreislauf einschließlich des Dialysatorfilters mit einer Flüssigkeit zu füllen (das sog. Priming), um die im extrakorporalen Kreislauf vorhandene Luft zu entfernen. Dazu wird vor allem Kochsalzlösung verwendet. Alternativ wird auch ultrareine Dialysierflüssigkeit verwendet und zwar insbesondere bei sog. Online-Maschinen mit zwei Dialysierflüssigkeitsfiltern und direktem Anschluss an eine zentrale Dialysierflüssigkeitsversorgung. Beim Befüllen mit Kochsalzlösung wird ein vorgefüllter Beutel mit Kochsalzlösung manuell an das arterielle Blutschlauchende angeschlossen. Die Blutpumpe der Dialysemaschine fördert dann die Flüssigkeit durch den extrakorporalen Blutkreislauf. Die Kochsalzlösung tritt schließlich am venösen Ende des Blutschlauches aus. Nach ausreichend umgewälzter Flüssigkeit ist der extrakorporale Kreislauf befüllt und damit für die Blutbehandlung vorbereitet.

Beim Befüllen der Blutseite des Dialysators muss die Luft durch Dialysierflüssigkeit bzw. Kochsalzlösung ersetzt werden. Bei diesem Vorgang entweichen viele einzelne Luftblasen, die aus dem Inneren der Fasern des Dialysators ausgespült werden müssen. Um diese Luft zu entfernen, werden verschiedene Verfahren angewandt. So kann der Dialysator manuell gedreht werden, kann gegen den Dialysator geklopft werden oder können bei einem automatischen Vorbereitungsprozedere Druckstöße erzeugt werden. Die entweichende Luft gelangt in den ausgansseitigen Blutschlauch des Dialysators und wird erst in der venösen Tropfkammer abgeschieden, was dort zu einer unerwünschten Pegelabsenkung führt. Diese Pegelabsenkung muss in einem weiteren Schritt korrigiert werden. Dialysatoren sind aus de Dokumenten US 2009/084720 A1, US 2015/367062 A1, DE 197 28 071 C2 bekannt.

Aufgabe der vorliegenden Erfindung ist es, einen Dialysator bzw. eine Dialysemaschine bereitzustellen, die eine einfachere, schnellere und/oder zuverlässigere Vorbereitung im Vorfeld einer Blutbehandlung ermöglichen.

Die vorliegende Erfindung beansprucht einen Dialysator gemäß Anspruch 1 und eine Dialysatorvorrichtung gemäß Anspruch 5. Die übrigen Ansprüche beziehen sich auf bevorzugte Ausführungsformen. Ein erfindungsgemäßer Dialysator für eine extrakorporale Blutbehandlung weist ein langgestrecktes, vorzugsweise zylinderförmiges Dialysatorgehäuse und zumindest eine Dialysemembran auf. Die Dialysemembran trennt einen Innenraum des Dialysatorgehäuses in eine mit zwei Dialysierflüssigkeitsanschlüssen (einem Dialysierflüssigkeitszuleitungsanschluss und einem Dialysierflüssigkeitsableitungsanschluss) versehene Dialysierflüssigkeitskammer und eine mit zwei Blutanschlüssen (einem Blutzuleitungsanschluss und einem Blutableitungsanschluss) versehene Blutkammer. Die Dialysierflüssigkeitsanschlüsse sind insbesondere als Hansen-Konnektoren ausgebildet und die Blutanschlüsse sind insbesondere als Luer-Konnektoren ausgebildet, oder mit dem Blutschlauchsystem verklebt.

Der Dialysator weist des Weiteren einen zusätzlichen Entlüftungsauslass zur Entlüftung der Blutkammer auf, der an oder nahe einer Stirnseite des Dialysatorgehäuses an diesem ausgebildet ist. Der Entlüftungsauslass ist mittels einer luftdurchlässigen Entlüftungsmembran verschlossen. Insbesondere kann diese Entlüftungsmembran wasserundurchlässig (hydrophob) ausgebildet sein. Durch den Entlüftungsauslass bzw. die Entlüftungsmembran kann zuvor in der Blutkammer vorhandene Luft beim Befüllen der Blutkammer mit einer wässrigen Flüssigkeit entweichen, bis die Blutkammer vollständig mit der Flüssigkeit befüllt ist. Die unterschiedliche Durchlässigkeit der Entlüftungsmembran gegenüber Luft und gegenüber wässriger Flüssigkeit wird durch die Größe von Poren, die in der Entlüftungsmembran vorgesehen sind, erreicht. Vorzugsweise haben diese Poren einen Durchmesser kleiner gleich 0,2 µm, damit die Entlüftungsmembran gleichzeitig als eine Sterilbarriere dient, welche das Eindringen von Keimen in das Blut verhindert. Insbesondere kann der Dialysator derart ausgebildet sein, dass (fluidmechanisch bzw. entlang von Strömungslinien) zwischen dem zusätzlichen Entlüftungsauslass und der Blutkammer bzw. zwischen der Entlüftungsmembran des zusätzlichen Entlüftungsauslasses und der Blutkammer keine Membran, insbesondere nicht die Dialysemembran, vorgesehen ist.

Durch das Vorsehen des Entlüftungsauslasses bzw. der Entlüftungsmembran wird ein direktes Entweichen der Luft aus der Blutkammer in die Umgebung des Dialysators ermöglicht. Dieses direkte Entweichen der Luft verkürzt die Strömungswege der entweichenden Luft und verringert somit die Wahrscheinlichkeit, dass einzelne gelöste Luftblasen beim Hinausspülen an anderen Oberflächen hängen bleiben. Des Weiteren führen die verkürzten Strömungswege zu einer schnelleren Luftentfernung. Schließlich wird auch das Absinken des Pegels in der venösen Tropfkammer zumindest verringert, so dass die Korrektur dieses Pegels zumindest weniger Zeit in Anspruch nimmt.

Der Entlüftungsauslass bzw. die Entlüftungsmembran ist bezüglich einer Blutströmungsrichtung im Blutableitungsanschluss zwischen einer Austrittsfläche des Blutableitungsanschlusses und dem Dialysierflüssigkeitszuleitungsanschluss vorgesehen.

Bei der Vorbereitung einer Dialysevorrichtung auf eine Blutbehandlung wird zunächst der Blutkreislauf mit Flüssigkeit befüllt, bevor der Dialysierflüssigkeitskreislauf mit einer Flüssigkeit befüllt wird. Das bedeutet, dass beim Füllen des Blutkreislaufs auch über die semipermeable, gegenüber größeren Eiweißmolekülen wie Albumin undurchlässige aber gegenüber wässrigen Lösungen und Luft durchlässige Dialysemembran Luft vom Blutkreislauf bzw. von der Blutkammer in den Dialysekreislauf bzw. die Dialysierflüssigkeitskammer entweichen kann. Dies gilt jedoch vor allem für Luft bzw. Luftbläschen, die in der Blutkammer zwischen einem Blutzuleitungsanschluss und dem Dialysierflüssigkeitszuleitungsanschluss verortet sind. Um auch zwischen dem Dialysierflüssigkeitszuleitungsanschluss und der Austrittsfläche des Blutableitungsanschluss verortete Luftbläschen möglichst schnell und zuverlässig zu entfernen, ist es vorteilhaft, den Entlüftungsauslass bzw. die Entlüftungsmembran in diesem Bereich der Blutkammer vorzusehen.

Das erfindungsgemäße Dialysatorgehäuse kann eine Dialysatorkappe aufweisen, an welcher der Entlüftungsauslass bzw. die Entlüftungsmembran vorgesehen ist. Bekannte Dialysatoren weißen ein Gehäuse mit einem mittigen, hohlzylinderförmigen Abschnitt und zwei jeweils an einer Stirnseite des hohlzylinderförmigen Abschnitts angeordnete Dialysatorkappen auf. Wird die Entlüftungsmembran an zumindest einer der Dialysatorkappe vorgesehen, ist es in vorteilhafter Weise möglich, die zur Herstellung der hohlzylinderförmigen Abschnitte verwendeten Werkzeuge weiter zu verwenden. Auch ist es auf einfache Weise möglich, den Dialysator nur durch Änderung der Dialysatorkappen entsprechend einer beabsichtigten Orientierung des Dialysators beim Betrieb in einer Dialysevorrichtung anzupassen.

Der Entlüftungsauslass bzw. die Entlüftungsmembran ist derart ausgebildet, dass er bzw. sie sich ringförmig um den Blutableitungsanschluss erstreckt. Somit kann unabhängig von der Orientierung des Dialysators um eine Erstreckungsachse des Blutableitungsanschlusses eine gleichbleibende Qualität der Luftentfernung aus dem Dialysator sichergestellt werden.

Die Entlüftungsmembran kann zumindest zweilagig ausgebildet sein. Eine, insbesondere der Blutkammer bzw. einem Inneren der Blutkammer zugewandte, erste Lage der Entlüftungsmembran kann hydrophob ausgebildet sein und eine, insbesondere von der Blutkammer abgewandte bzw. einer Umgebung zugewandte, zweite Lage der Entlüftungsmembran kann als Sterilbarriere ausgebildet sein. Insbesondere kann die erste Lage von der zweiten Lage beabstandet sein. Durch diese Zweiteilung kann die Entlüftungsmembran auf einfache Weise bezüglich ihrer Wasserundurchlässigkeit einerseits und bezüglich ihrer Sterilbarriereeigenschaft andererseits optimiert werden.

Insbesondere kann die erste Lage der Entlüftungsmembran größere Poren und eine kleinere Oberfläche als die zweite Lage der Entlüftungsmembran aufweisen. Anders ausgedrückt kann die Fläche der zweiten Lage größer sein als die Fläche der ersten Lage. Insbesondere können sich die Poren der ersten Lage und die Poren der zweiten Lage überlappen. Wird die Oberfläche der als Sterilbarriere dienenden zweiten Lage größer ausgebildet als die für die Wasserundurchlässigkeit verantwortliche erste Lage, kann trotz der kleineren Poren in der zweiten Lage ein hoher Druckverlust vermieden werden.

Die vorliegende Erfindung betrifft auch eine Dialysevorrichtung, die mit einem erfindungsgemäßen Dialysator ausgestattet ist.

Der Dialysator kann derart an der Dialysevorrichtung angebracht sein, dass der Entlüftungsauslass bzw. die Entlüftungsmembran bei betriebsbereiter Aufstellung der Dialysevorrichtung in Erdbeschleunigungsrichtung zumindest abschnittsweise oberhalb des Dialysierflüssigkeitszuleitungsanschlusses angeordnet ist.

Wie oben erläutert, können Luftbläschen, welche vor dem Primen in der Blutkammer zwischen einem Blutzuleitungsanschluss und dem Dialysierflüssigkeitszuleitungsanschluss vorhanden sind, auch über die Dialysemembran entweichen. Um auch Luftbläschen zwischen dem Dialysierflüssigkeitszuleitungsanschluss und der Austrittsfläche des Blutableitungsanschluss zu entfernen, ist es vorteilhaft, die Entlüftungsmembran in diesem Bereich der Blutkammer vorzusehen.

Der Dialysator kann derart an der Dialysevorrichtung angebracht sein, dass der Entlüftungsauslass bzw. die Entlüftungsmembran bei betriebsbereiter Aufstellung der Dialysevorrichtung in Erdbeschleunigungsrichtung zumindest abschnittsweise oberhalb der Blutkammer angeordnet ist.

Wird der Entlüftungsauslass bzw. die Entlüftungsmembran zumindest abschnittsweise oberhalb der Blutkammer angeordnet, ist eine zumindest nahezu restlose Entfernung der Luft aus der Blutkammer möglich.

Eine offenbarungsgemäße, nicht beanspruchte Dialysevorrichtung kann alternativ auch einen gattungsgemäßen Dialysator für eine extrakorporale Blutbehandlung aufweisen, welcher versehen ist mit einem langgestreckten, vorzugsweise zylinderförmigen Dialysatorgehäuse, zumindest einer Dialysemembran, welche einen Innenraum des Dialysatorgehäuses in eine mit einem Dialysierflüssigkeitszuleitungsanschluss sowie einem Dialysierflüssigkeitsableitungsanschluss versehene Dialysierflüssigkeitskammer und eine mit einem Blutzuleitungsanschluss sowie einem Blutableitungsanschluss versehene Blutkammer trennt. Eine derart ausgestattete Dialysevorrichtung zeichnet sich aus durch eine wasserundurchlässige und luftdurchlässige Entlüftungsmembran, welche in zumindest einem Abschnitt eine Umwandung des Blutableitungsschlauchs bildet. Somit kann wiederum beim Befüllen der Blutkammer mit einer Flüssigkeit zuvor in der Blutkammer vorhandene Luft durch die Entlüftungsmembran entweichen, bis die Blutkammer vollständig mit der Flüssigkeit befüllt ist. Die Entlüftungsmembran der erfindungsgemäßen Dialysevorrichtung weist Poren auf, deren Durchmesser vorzugsweise jeweils kleiner gleich 0,2 µm sind, um als Sterilbarriere dienen zu können.

Ist die Entlüftungsmembran in der Umwandung des an dem Dialysator angeschlossenen Blutableitungsschlauchs vorgesehen, kann eine verbesserte Entlüftung eines herkömmlichen Dialysators erreicht werden.

Die erfindungsgemäße Dialysevorrichtung kann derart ausgebildet sein, dass der entsprechende Dialysator derart an der Dialysevorrichtung angebracht ist, dass eine durch die Blutkammer geleitete Flüssigkeit diametral entgegen der Erdbeschleunigung fließt. Anders ausgedrückt kann der Dialysator derart an der Dialysevorrichtung angebracht sein, dass sich dieser in vertikaler Richtung erstreckt, wenn die Dialysevorrichtung aufrecht auf einer horizontalen Ebene gelagert ist.

Wird zur Entfernung von Luftbläschen in der Blutkammer deren Auftriebskraft gegenüber Wasser im Erdanziehungsfeld zu Nutze gemacht, kann eine zuverlässigere und schnellere Luftentfernung erreicht werden.

Der Dialysator kann alternativ auch derart an der Dialysevorrichtung angebracht sein, dass eine durch die Blutkammer geleitete Flüssigkeit senkrecht zur Erdbeschleunigung fließt. Anders ausgedrückt kann der Dialysator derart an der Dialysevorrichtung angebracht sein, dass sich dieser in horizontaler Richtung erstreckt, wenn die Dialysevorrichtung aufrecht auf einer horizontalen Ebene gelagert ist.

Kurzbeschreibung der Zeichnungen

Die vorliegende Erfindung wird im Folgenden anhand bevorzugter Ausführungsbeispiele unter Bezugnahme auf die beigefügten Zeichnungen näher beschrieben. Es zeigen:
Fig. 1 eine perspektivische Schemazeichnung einer Dialysevorrichtung mit vertikal angeordnetem Dialysator;
Fig. 2 eine Schnittzeichnung eines Dialysators gemäß einer ersten Ausführungsform;
Fig. 3 eine Detailansicht einer Blutauslassseite des in Fig. 2 gezeigten Dialysators; Dieser Dialysator ist nicht Gegenstand der vorliegenden Erfindung, wie sie in den Ansprüchen definiert ist.
Fig. 4 eine der Fig. 3 entsprechende Detailansicht einer Blutauslassseite eines Dialysators gemäß einer zweiten Ausführungsform Dieser Dialysator ist nicht Gegenstand der vorliegenden Erfindung, wie sie in den Ansprüchen definiert ist.
Fig. 5 eine der Fig. 3 entsprechende Detailansicht einer Blutauslassseite eines erfindungsgemäßen Dialysators gemäß einer dritten Ausführungsform zusammen mit einer Frontalansicht;
Fig. 6 eine perspektivische Schemazeichnung einer gattungsgemäßen Dialysevorrichtung mit horizontal angeordnetem Dialysator;
Fig. 7 eine Schnittzeichnung eines Dialysators gemäß einer vierten Ausführungsform; Dieser Dialysator ist nicht Gegenstand der vorliegenden Erfindung, wie sie in den Ansprüchen definiert ist.
Fig. 8 eine Detailansicht einer Blutauslassseite des in Fig. 7 gezeigten Dialysators; Dieser Dialysator ist nicht Gegenstand der vorliegenden Erfindung, wie sie in den Ansprüchen definiert ist. und
Fig. 9 eine der Fig. 3 entsprechende Detailansicht einer Blutauslassseite eines Dialysators gemäß einer fünften Ausführungsform Dieser Dialysator ist nicht Gegenstand der vorliegenden Erfindung, wie sie in den Ansprüchen definiert ist.

Gleiche oder funktional äquivalente Merkmale sind in den einzelnen Figuren mit denselben Bezugszeichen versehen, wobei die Bezugszeichen der zweiten, dritten, vierten und fünften Ausführungsform mit ...', ...", ...‴ und ... "" gekennzeichnet sind.

### Detaillierte Beschreibung bevorzugter Ausführungsformen

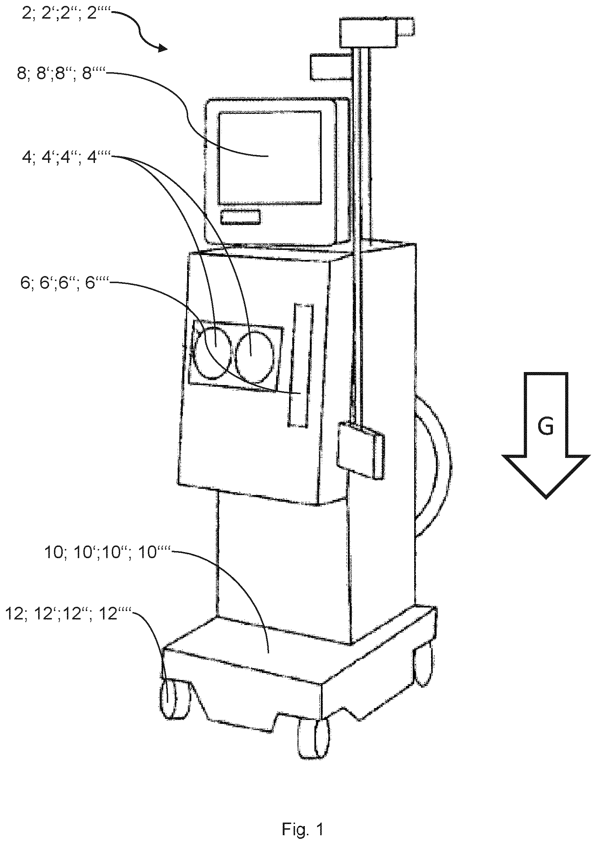

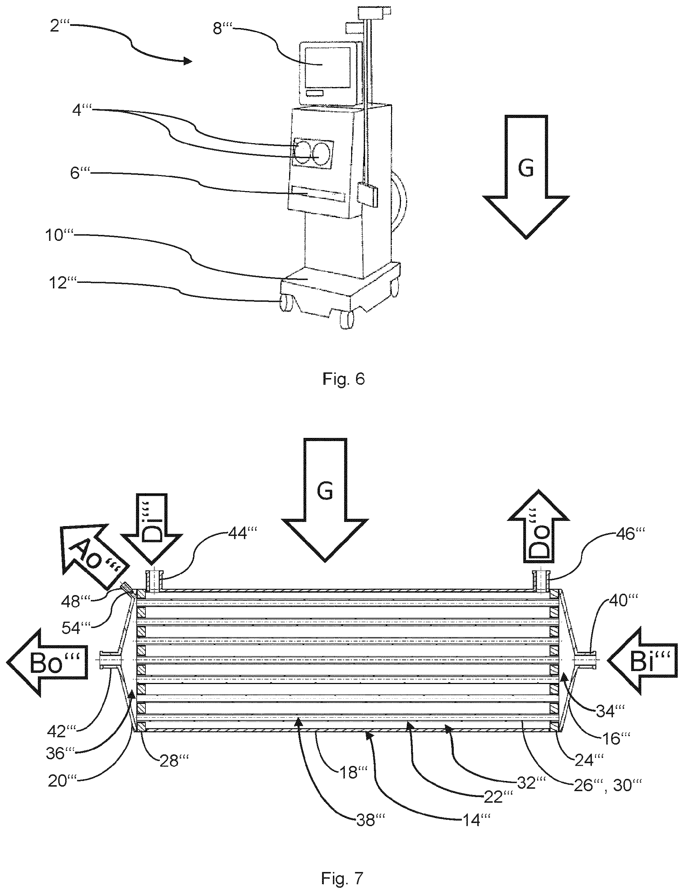

Eine gattungsgemäße Dialysevorrichtung 2; 2'; 2"; 2ʺʺ weist an einer Vorderseite Peristaltikpumpen 4; 4'; 4"; 4ʺʺ zum Fördern von Blut und Dialysierflüssigkeit auf. In der Nähe der Pumpen 4; 4'; 4"; 4ʺʺ ist ein Dialysator 6; 6'; 6"; 6ʺʺ vorgesehen. Im Betrieb ist der Dialysator 6; 6'; 6"; 6ʺʺ über (nicht gezeigte) Schläuche einerseits mit der Dialysevorrichtung 2; 2'; 2"; 2ʺʺ und andererseits mit einem Patienten verbunden. Eine der Pumpen 4; 4'; 4"; 4ʺʺ fördert zu reinigendes Blut zum Dialysator 6; 6'; 6"; 6ʺʺ. Die andere der beiden Pumpen 4; 4'; 4"; 4ʺʺ fördert Dialysierflüssigkeit zum Dialysator 6; 6'; 6"; 6"". Um die Dialysevorrichtung 2; 2'; 2"; 2ʺʺ bedienen zu können ist an ihrer Vorderseite ein vorzugsweise als Touchscreen ausgebildeter Bildschirm 8; 8'; 8"; 8ʺʺ vorgesehen. Damit die Dialysevorrichtung 2; 2'; 2"; 2ʺʺ auf einer horizontalen Ebene einen stabilen und aufrechten Stand hat, weist sie an einer Unterseite ein Basisteil 10; 10'; 10"; 10ʺʺ auf. Um gleichwohl einfach transportabel zu sein, sind an einer Unterseite des Basisteils 10; 10'; 10"; 10ʺʺ Lenkrollen 12; 12'; 12"; 12ʺʺ vorgesehen. Bei der in Figur 1 gezeigten Dialysevorrichtung 2; 2'; 2"; 2ʺʺ ist der Dialysator 6; 6'; 6"; 6ʺʺ vertikal angebracht. Wie in Figur 6 gezeigt ist, kann die Dialysemaschine 2‴ auch derart ausgebildet werden, dass sich der Dialysator 6‴ horizontal erstreckt.

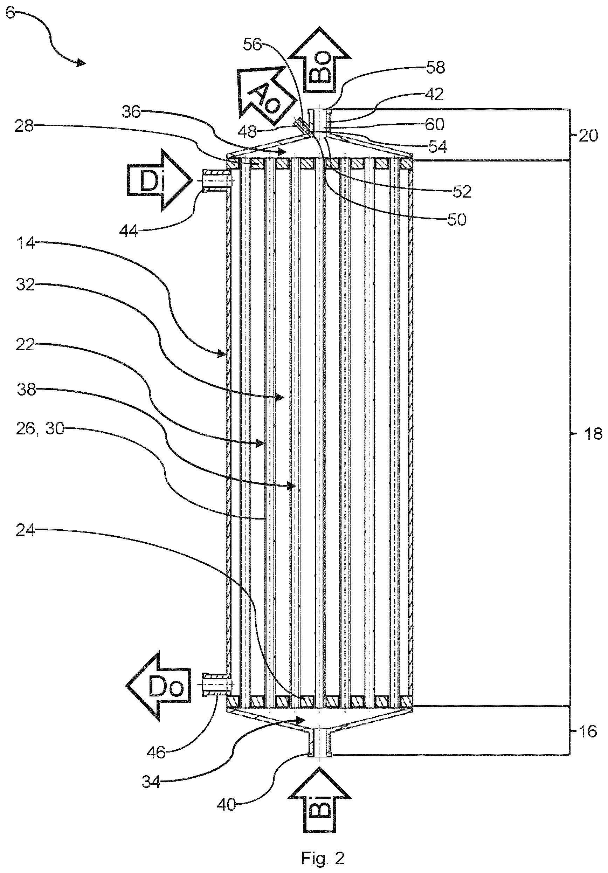

In Figur 2 wird eine Schnittzeichnung eines Dialysators 6 gemäß einer ersten Ausführungsform gezeigt. Der Dialysator 6 weist ein Dialysatorgehäuse 14 auf, welches in eine deckelförmige Bluteinlass-Dialysatorkappe 16, einen im Wesentlichen zylinderförmigen, insbesondere kreiszylindrischen, Dialysierflüssigkeitsteil 18 und in eine ebenfalls deckelförmigen Blutauslass-Dialysatorkappe 20 untergliedert werden kann.

In dem Dialysierflüssigkeitsteil 18 des Dialysatorgehäuses 14 ist ein Hohlfaserbündelteil 22 eingebettet. Das Hohlfaserbündelteil 22 ist gebildet aus einer ersten, insbesondere runden, Hohlfaserbündelfassung 24, in der die einen Enden von Hohlfasern 26 eingegossen sind, einem Hohlfaserbündel, das aus den parallel zueinander angeordneten Hohlfasern 26 besteht, und aus einer zweiten, insbesondere runden, Hohlfaserbündelfassung 28, in der die anderen Enden der Hohlfasern 26 eingegossen sind. Das Hohlfaserfiltermodul 22 wird also an den Enden vergossen, mit dem Verguss gefasst und gegebenenfalls nach dem Vergießen an den beiden Enden abgeschnitten, um somit versehentlich zugegossene Hohlfasern 26 wieder zugänglich zu machen. Die Umwandungen der einzelnen Hohlfasern 26 stellen eine Dialysemembran 30 dar. Die Dialysemembran 30 weist Poren auf, deren Größe so abgestimmt ist, dass die Dialysemembran 30 gegenüber Wasser durchlässig, gegenüber größeren Eiweißmolekülen wie Albumin jedoch undurchlässig ist.

Um den zwischen den beiden Hohlfaserbündelfassungen 24 und 28 aufgespannten Raum des Hohlfaserbündelteils 22 erstreckt sich der insbesondere kreiszylinderschalenförmige Dialysierflüssigkeitsteil 18 des Dialysatorgehäuses 14. Der Raum zwischen den Hohlfasern 26, der von den beiden Hohlfaserbündelfassungen 24 und 28 sowie dem Dialysierflüssigkeitsteil 18 des Dialysatorgehäuses 4 umfasst wird, entspricht einer Dialysierflüssigkeitskammer 32.

Die deckelförmige Bluteinlass-Dialysatorkappe 16 des Dialysatorgehäuses 14 ist derart an der ersten Hohlfaserbündelfassung 24 auf deren vom Hohlfaserbündelteil 22 abgewandten Seite angeordnet oder angebracht, dass zwischen der Bluteinlass-Dialysatorkappe 16 und der ersten Hohlfaserbündelfassung 24 ein Blutverteilerraum 34 definierbar ist.

Die deckelförmige Blutauslass-Dialysatorkappe 20 des Dialysatorgehäuses 14 ist derart an der zweiten Hohlfaserbündelfassung 28 auf deren vom Hohlfaserbündelteil 22 abgewandten Seite angeordnet oder angebracht, dass zwischen der Blutauslass-Dialysatorkappe 20 und der zweiten Hohlfaserbündelfassung 28 ein Blutsammelraum 36 definierbar ist.

Der Blutverteilerraum 34, der Raum im Inneren der Hohlfasern 26 und der Blutsammelraum 36 entsprechen zusammen einer Blutkammer 38 des Dialysators 6.

An der Bluteinlass-Dialysatorkappe 16 des Dialysatorgehäuses 14 ist ein Blutzuleitungsanschluss 40 zum Einleiten von Blut vorgesehen (siehe Pfeil "Bi" in Figur 2). An der Blutauslass-Dialysatorkappe 20 des Dialysatorgehäuses 14 ist ein Blutableitungsanschluss 42 zum Ableiten von Blut vorgesehen (siehe Pfeil "Bo" in Figur 2). Am Dialysierflüssigkeitsteil 18 des Dialysatorgehäuses 14 ist in der Nähe der zweiten Hohlfaserbündelfassung 28 ein Dialysierflüssigkeitszuleitungsanschluss 44 zum Einleiten von Dialysierflüssigkeit in die Dialysierflüssigkeitskammer 32 vorgesehen (siehe Pfeil "Di" in Figur 2). In der Nähe der ersten Hohlfaserbündelfassung 24 ist am Dialysierflüssigkeitsteil 18 des Dialysatorgehäuses 14 ein Dialysierflüssigkeitsableitungsanschluss 46 zu Ableiten von Dialysierflüssigkeit vorgesehen (siehe Pfeil "Do" in Figur 2).

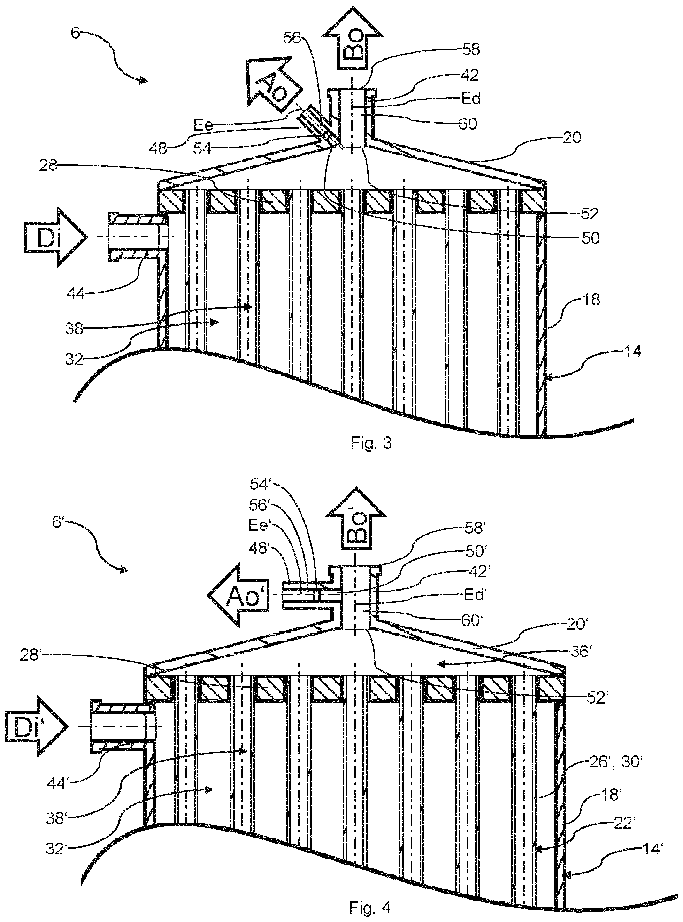

Wie in Figur 3 genauer gezeigt ist, weist die Blutauslass-Dialysatorkappe 20 einen Entlüftungsauslass 48 auf. Der Entlüftungsauslass 48 hat die Form eines

Stutzens bzw. eines Hohlzylinders und erstreckt sich gegenüber der Erstreckungsrichtung Ed des Dialysators 6 in einer angewinkelten, zum Blutableitungsanschluss 42 zugeneigten Achse Ee. Eine Mündung 50 des Entlüftungsauslasses 48 ist unmittelbar zu einer Mündung 52 des Blutableitungsanschlusses 42 benachbart. Innerhalb des Entlüftungsauslasses 48 ist eine Entlüftungsmembran 54 vorgesehen, welche einen im Inneren des Entlüftungsauslasses 48 entlang dessen Erstreckungsrichtung Ee verlaufenden Kanal 56 luftdurchlässig und wasserundurchlässig verschließt.

Der in den Figuren 2 und 3 gezeigte Dialysator 6 ist dafür vorgesehen, an eine Dialysevorrichtung 2 derart vertikal angebracht zu werden, dass sich der Blutzuleitungsanschluss 40 unten und der Blutableitungsanschluss 42 oben befindet.

Um in der Blutkammer 38 vorhandene Luft vor einer Blutbehandlung zu entfernen, wird der Blutableitungsanschluss 42 bzw. ein an den Blutableitungsanschluss 42 angebrachter (nicht gezeigter) Schlauch geschlossen bzw. abgeklemmt und wird Kochsalzlösung über den Blutzuleitungsanschluss 40 der Blutkammer 38 zugeführt. Durch das Befüllen der Blutkammer 38 mit Kochsalzlösung wird die zuvor in der Blutkammer 38 befindliche Luft nach und nach über den Entlüftungsauslass 48 aus der Blutkammer 38 entlassen (siehe Pfeil "Ao" in Figur 2). Bis zum Erreichen der zweiten Hohlfaserbündelfassung 28 wird Luft auch zu einem großen Teil über die Dialysemembran 30 in die Dialysierflüssigkeitskammer 32 verdrängt. Sobald der Pegel der Kochsalzlösung die zweite Hohlfaserbündelfassung 28 erreicht hat, erfolgt die Entlüftung zum überwiegenden Teil über den Entlüftungsauslass 48. Sobald der Pegel der Kochsalzlösung die Entlüftungsmembran 54 überstiegen hat, erfolgt die Entlüftung nicht mehr über Verdrängung sondern nur noch über Diffusion.

Um einen im Vergleich zur ersten Ausführungsform noch größeren Teil der in der Blutkammer 38 vorhandenen Luft über die schneller erfolgende Verdrängung zu entfernen, wird bei einem Dialysator 6' gemäß einer in Figur 4 gezeigten zweiten Ausführungsform ein Entlüftungsauslass 48' noch näher an einer Austrittsfläche 58' angeordnet. Eine Mündung 50' des Entlüftungsauslasses 48' steht somit lediglich in Kontakt mit einem im Inneren des Blutableitungsanschluss 42 verlaufenden Kanal 60'. Damit das Längsausmaß des Dialysators 6' gemäß der zweiten Ausführungsform nicht größer ist als das Längsausmaß des Dialysators 6 gemäß der ersten Ausführungsform, ist der Entlüftungsauslass 48' in Form eines Stutzens bzw. eines Hohlzylinders ausgebildet, der sich in einer gegenüber einer Erstreckungsrichtung Ed' des Dialysators 6' rechtwinklig angeordneten Achse Ee' erstreckt. Wie der Entlüftungsauslass 6 gemäß der ersten Ausführungsform weist auch der Entlüftungsauslass 6" gemäß der zweiten Ausführungsform eine Entlüftungsmembran 54' auf, welche einen im Inneren des Entlüftungsauslasses 48' entlang dessen Erstreckungsrichtung Ee' verlaufenden Kanal 56' luftdurchlässig und wasserundurchlässig verschließt.

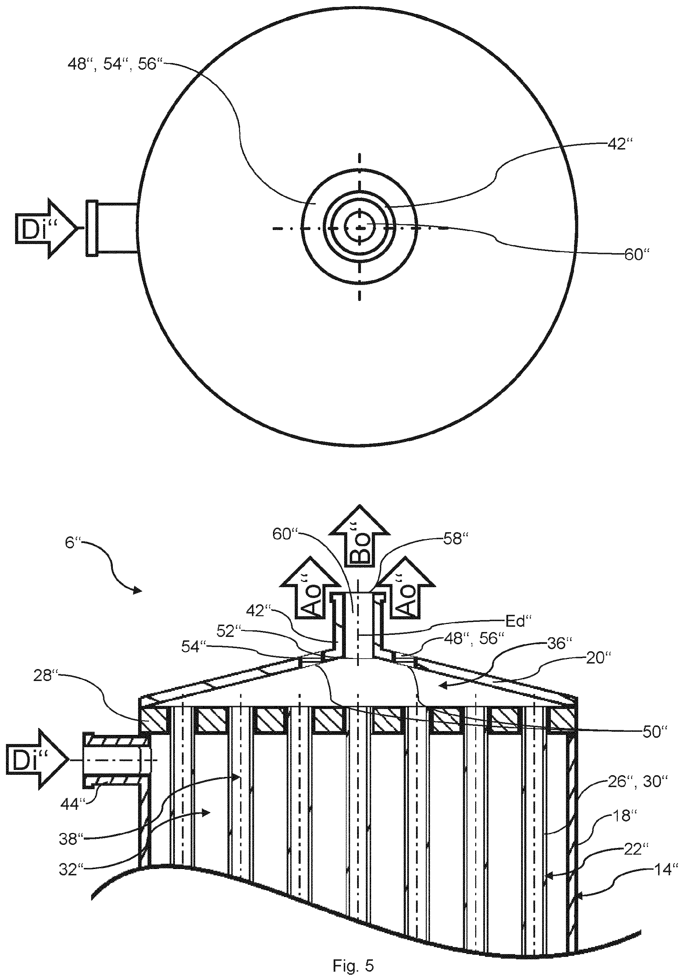

Gemäß einer in Figur 5 gezeigten dritten Ausführungsform eines Dialysators gemäß Erfindung : Ein 6" Entlüftungsauslass 48" ist derart an einer Blutauslass-Dialysatorkappe 20" ausgebildet, dass er sich ringförmig um einen Blutableitungsanschluss 42" erstreckt. Damit die strukturelle Integrität der Blutauslass-Dialysatorkappe 20" gewährleitet wird, kann der ringförmige Entlüftungsauslass 48" auch mehrerer mittels (nicht gezeigter) ringförmig um den Blutableitungsanschluss 42" angeordneter Entlüftungsauslässe umgesetzt sein oder können in dem Entlüftungsauslass 48" (nicht gezeigte) Stege zur Überbrückung einer entsprechenden Ringnut vorgesehen sein. In dem Entlüftungsauslass 48" ist eine Entlüftungsmembran 54" vorgesehen.

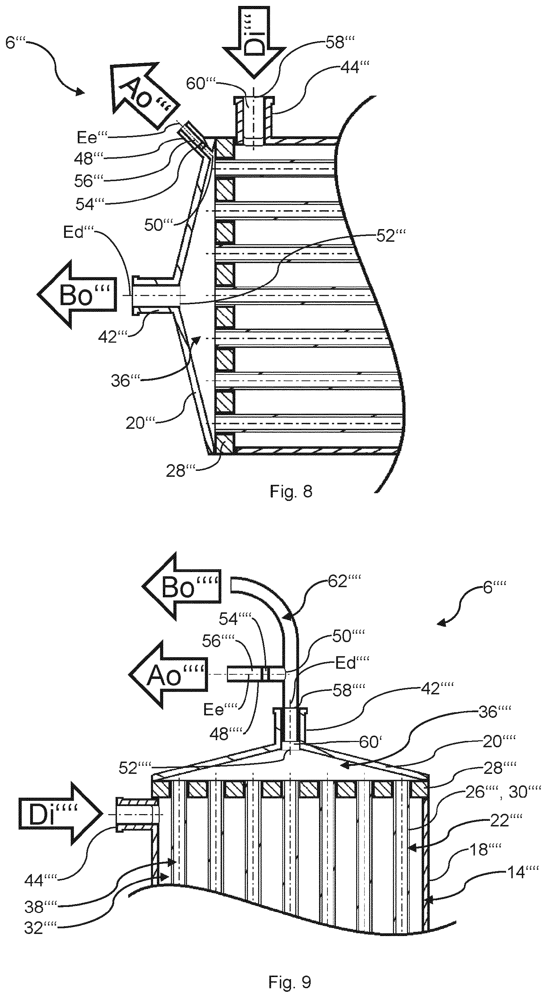

In den Figuren 7 und 8 ist ein Dialysator 6‴ gemäß einer vierten Ausführungsform gezeigt. Der Dialysator 6‴ ist dafür vorgesehen, wie in Figur 6 gezeigt, horizontal an eine Dialysevorrichtung 2 angebracht zu werden. Ein mit einer Entlüftungsmembran 54‴ ausgestatteter Entlüftungsauslass 48‴ ist am Rand einer Blutauslass-Dialysatorkappe 20‴ angeordnet. Somit wird gewährleitet, dass beim Befüllen des horizontal montierten Dialysators 6‴ die Entlüftungsmembran 54‴ möglichst spät benetzt wird.

In Figur 9 ist ein gattungsgemäßer Dialysator 6ʺʺ gezeigt. An einem Blutableitungsanschluss 42ʺʺ des Dialysators 6ʺʺ ist ein Schlauch 62ʺʺ angeschlossen, der in der Nähe des Blutableitungsanschlusses 42ʺʺ einen Entlüftungsauslass 48ʺʺ mit einer Entlüftungsmembran 54ʺʺ aufweist.

### Bezugszeichenliste

- 2, 2', 2", 2‴, 2"": Dialysevorrichtung
- 4, 4', 4", 4‴, 4ʺʺ: Peristaltikpumpe
- 6, 6', 6", 6‴, 6ʺʺ: Dialysator
- 8, 8', 8", 8‴, 8ʺʺ: Bildschirm
- 10, 10', 10", 10‴, 10ʺʺ: Basisteil
- 12, 12', 12", 12‴, 12ʺʺ: Lenkrollen
- 14, 14', 14", 14‴, 14"": Dialysatorgehäuse
- 16, 16‴: Bluteinlass-Dialysatorkappe
- 18, 18', 18", 18‴, 18ʺʺ: Dialysierflüssigkeitsteil
- 20, 20', 20", 20‴, 20ʺʺ: Blutauslass-Dialysatorkappe
- 22, 22', 22", 22"', 22ʺʺ: Hohlfaserbündelteil
- 24, 24‴: erste Hohlfaserbündelfassung
- 26, 26', 26", 26‴, 26ʺʺ: Hohlfaser
- 28, 28', 28", 28"', 28ʺʺ: zweite Hohlfaserbündelfassung
- 30, 30', 30", 30"', 30ʺʺ: Dialysemembran
- 32, 32', 32", 32‴, 32ʺʺ: Dialysierflüssigkeitskammer
- 34, 34‴: Blutverteilerraum
- 36, 36', 36", 36‴, 36ʺʺ: Blutsammelraum
- 38, 38', 38", 38‴, 38ʺʺ: Blutkammer
- 40, 40‴: Blutzuleitungsanschluss
- 42, 42', 42", 42‴, 42ʺʺ: Blutableitungsanschluss
- 44, 44', 44", 44‴, 44ʺʺ: Dialysierflüssigkeitszuleitungsanschluss
- 46, 46‴: Dialysierflüssigkeitsableitungsanschluss
- 48, 48', 48", 48‴, 48ʺʺ: Entlüftungsauslass
- 50, 50', 50", 50‴, 50ʺʺ: Mündung des Entlüftungsauslasses
- 52, 52', 52", 52‴, 52ʺʺ: Mündung des Blutableitungsanschlusses
- 54, 54', 54", 54‴, 54ʺʺ: Entlüftungsmembran
- 56, 56', 56", 56‴, 56ʺʺ: Kanal im Entlüftungskanal
- 58, 58', 58", 58‴, 58ʺʺ: Austrittsfläche des Blutableitungsanschlusses
- 60, 60', 60", 60‴, 60ʺʺ: Kanal im Blutableitungsanschluss
- 62ʺʺ: An Blutableitungsanschluss angeschlossener Schlauch
- Ao, Ao', Ao", Ao‴, Aoʺʺ: Luftströmung aus dem Entlüftungsauslass
- Bi, Bi‴: Strömung in die Blutkammer
- Bo, Bo', Bo", Bo‴, Boʺʺ: Strömung aus der Blutkammer
- Di, Di', Di", Di‴, Diʺʺ: Strömung in die Dialysierflüssigkeitskammer
- Do, Do‴: Strömung aus der Dialysierflüssigkeitskammer
- Ed, Ed', Ed", Ed‴, Edʺʺ: Erstreckungsachse des Dialysators
- Ee, Ee', Ee‴, Eeʺʺ: Erstreckungsachse des Entlüftungsauslasses
- G: Richtung der Erdbeschleunigung

## Patentansprüche

1. Dialysator (6") für eine extrakorporale Blutbehandlung mit
- einem langgestreckten, vorzugsweise zylinderförmigen Dialysatorgehäuse (14"),
- zumindest einer Dialysemembran (30"), welche einen Innenraum des Dialysatorgehäuses (14") in eine mit einem Dialysierflüssigkeitszuleitungsanschluss (44") sowie einem Dialysierflüssigkeitsableitungsanschluss versehene Dialysierflüssigkeitskammer (32') und eine mit einem Blutzuleitungsanschluss sowie einem Blutableitungsanschluss (42") versehene Blutkammer (38") trennt, und
- einem zusätzlichen Entlüftungsauslass (48") zur Entlüftung der Blutkammer (38"), der an oder nahe einer Stirnseite des Dialysatorgehäuses (14") an diesem ausgebildet ist,
**wobei** der zusätzliche Entlüftungsauslass (48") bezüglich einer Blutströmungsrichtung (Bo") im Blutableitungsanschluss (42") zwischen einer Austrittsfläche (58") des Blutableitungsanschlusses (42") und dem Dialysierflüssigkeitszuleitungsanschluss (44") vorgesehen ist,
**dadurch gekennzeichnet, dass**
der zusätzliche Entlüftungsauslass (48") ringförmig um den Blutableitungsanschluss (42") ausgebildet ist,
der zusätzliche Entlüftungsauslass (48") mittels einer luftdurchlässigen Entlüftungsmembran (54") verschlossen ist.

2. Dialysator (6") gemäß Anspruch 1, **dadurch gekennzeichnet, dass** das Dialysatorgehäuse (14") eine Dialysatorkappe (20") aufweist, an welcher der zusätzliche Entlüftungsauslass (48") vorgesehen ist.

3. Dialysator (6; 6'; 6"; 6‴) gemäß Anspruch 1 oder 2, **dadurch gekennzeichnet, dass** die Entlüftungsmembran (54") zumindest zweilagig ausgebildet ist, eine erste Lage der Entlüftungsmembran (54") hydrophob ausgebildet ist und eine zweite Lage der Entlüftungsmembran (54") als Sterilbarriere ausgebildet ist.

4. Dialysator (6; 6'; 6"; 6‴) gemäß Anspruch 3, **dadurch gekennzeichnet, dass** die erste Lage der Entlüftungsmembran (54") größere Poren und eine kleinere Oberfläche als die zweite Lage der Entlüftungsmembran (54") aufweist.

5. Dialysevorrichtung für eine extrakorporale Blutbehandlung mit einem Dialysator (6") gemäß Anspruch 1.

6. Dialysevorrichtung gemäß Anspruch 5, **dadurch gekennzeichnet, dass** der Dialysator (6") derart an der Dialysevorrichtung angebracht ist, dass der zusätzliche Entlüftungsauslass (48") bei betriebsbereiter Aufstellung der Dialysevorrichtung in Erdbeschleunigungsrichtung (G) zumindest abschnittsweise oberhalb des Dialysierflüssigkeitszuleitungsanschlusses (44") angeordnet ist.

7. Dialysevorrichtung gemäß Anspruch 5, **dadurch gekennzeichnet, dass** der Dialysator (6") derart an der Dialysevorrichtung angebracht ist, dass der Entlüftungsauslass (48") bei betriebsbereiter Aufstellung der Dialysevorrichtung in Erdbeschleunigungsrichtung (G) zumindest abschnittsweise oberhalb der Blutkammer (38") angeordnet ist.

8. Dialysevorrichtung gemäß Anspruch 6 oder 7, **dadurch gekennzeichnet, dass** der Dialysator (6") derart an der Dialysevorrichtung angebracht ist, dass eine durch die Blutkammer (38") geleitete Flüssigkeit diametral entgegen der Erdbeschleunigung (G) fließt.

9. Dialysevorrichtung gemäß Anspruch 5, **dadurch gekennzeichnet, dass** der Dialysator derart an der Dialysevorrichtung angebracht ist, dass eine durch die Blutkammer geleitete Flüssigkeit senkrecht zur Erdbeschleunigung (G) fließt.

## Claims

1. A dialyzer (6") for an extracorporeal blood treatment, comprising
- an elongated, preferably cylindrical dialyzer housing (14"),
- at least one dialysis membrane (30") which separates an internal space of the dialyzer housing (14") into a dialysis liquid chamber (32') provided with a dialysis liquid supply port (44") as well as a dialysis liquid discharge port and into a blood chamber (38") provided with a blood supply port as well as a blood discharge port (42"), and
- an additional ventilation outlet (48") for ventilating the blood chamber (38") formed on the dialyzer housing (14") on or close to an end face thereof,
**wherein** the additional ventilation outlet (48") is provided with respect to a blood flow direction (Bo") in the blood discharge port (42") between an exit area (58") of the blood discharge port (42") and the dialysis liquid supply port (44"),
**characterized in that**
the additional ventilation outlet (48") is formed in a ring shape around the blood discharge port (42"),
the additional ventilation outlet (48") is sealed by an air-permeable ventilation membrane (54").

2. The dialyzer (6") according to claim 1, **characterized in that** the dialyzer housing (6") comprises a dialyzer cap (20") on which the additional ventilation outlet (48") is provided.

3. The dialyzer (6; 6'; 6"; 6‴) according to claim 1 or 2, **characterized in that** the ventilation membrane (54") is formed with at least two layers, a first layer of the ventilation membrane (54") is hydrophobic, and a second layer of the ventilation membrane (54") is formed as a sterile barrier.

4. The dialyzer (6; 6'; 6"; 6‴) according to claim 3, **characterized in that** the first layer of the ventilation membrane (54") has larger pores and a smaller surface area than the second layer of the ventilation membrane (54").

5. A dialysis device for an extracorporeal blood treatment with a dialyzer (6") according to claim 1.

6. The dialysis device according to claim 5, **characterized in that**
the dialyzer (6") is attached to the dialysis device in such a manner that the additional ventilation outlet (48"), when the dialysis device is installed ready for operation, is arranged so as to be at least partially above the dialysis liquid supply port (44") in the direction of gravitational acceleration (G).

7. The dialysis device according to claim 5, **characterized in that** the dialyzer (6") is attached to the dialysis device in such a manner that the ventilation outlet (48"), when the dialysis device is installed ready for operation, is arranged so as to be at least partially above the blood chamber (38") in the direction of gravitational acceleration.

8. The dialysis device according to claim 6 or 7, **characterized in that** the dialyzer (6") is attached to the dialysis device in such a manner that a liquid conveyed through the blood chamber (38") flows diametrically against the gravitational acceleration (G).

9. The dialysis device according to claim 5, **characterized in that** the dialyzer is attached to the dialysis device in such a manner that a liquid conveyed through the blood chamber flows perpendicular to the gravitational acceleration (G).

## Revendications

1. Dialyseur (6") pour un traitement extracorporel du sang avec
- un boîtier de dialyseur (14") allongé, de préférence cylindrique,
- au moins une membrane de dialyse (30") qui sépare un espace interne du boîtier de dialyseur (14") en une chambre à liquide de dialyse (32") dotée d'un raccord d'alimentation en liquide de dialyse (44") ainsi que d'un raccord d'évacuation de liquide de dialyse et une chambre à sang (38") dotée d'un raccord d'alimentation en sang ainsi que d'un raccord d'évacuation du sang (42"), et
- une sortie d'aération supplémentaire (48") destinée à l'aération de la chambre à sang (38") qui est réalisée au niveau de celle-ci au niveau ou près d'une face avant du boîtier de dialyseur (14"),
**dans lequel** la sortie d'aération supplémentaire (48") est prévue par rapport à une direction d'écoulement du sang (Bo") dans le raccord d'évacuation du sang (42") entre une surface de sortie (58") du raccord d'évacuation du sang (42") et le raccord d'alimentation en liquide de dialyse (44"),
**caractérisé en ce que**
la sortie d'aération supplémentaire (48") est réalisée de manière annulaire autour du raccord d'évacuation du sang (42"),
la sortie d'aération supplémentaire (48") est fermée au moyen d'une membrane d'aération perméable à l'air (54").

2. Dialyseur (6") selon la revendication 1, **caractérisé en ce que** le boîtier de dialyseur (14") présente un capuchon de dialyseur (20) au niveau duquel la sortie d'aération supplémentaire (48") est prévue.

3. Dialyseur (6; 6'; 6"; 6‴) selon la revendication 1 ou **2,caractérisé en ce que** la membrane d'aération (54") est réalisée au moins en deux couches, une première couche de la membrane d'aération (54") est réalisée de manière hydrophobe et une seconde couche de la membrane d'aération (54") est réalisée en tant que barrière stérile.

4. Dialyseur (6 ; 6' ; 6" ; 6‴) selon la revendication 3, **caractérisé en ce que** la première couche de la membrane d'aération (54") présente des pores plus gros et une surface plus petite que la seconde couche de la membrane d'aération (54").

5. Dispositif de dialyse pour un traitement extracorporel du sang avec un dialyseur (6") selon la revendication 1.

6. Dispositif de dialyse selon la revendication 5, **caractérisé en ce que** le dialyseur (6") est placé au niveau du dispositif de dialyse de telle sorte que la sortie d'aération supplémentaire (48") est disposée au moins par sections au-dessus du raccord d'alimentation en liquide de dialyse (44") lorsque le dispositif de dialyse est en position de fonctionnement dans la direction d'accélération gravitationnelle (G).

7. Dispositif de dialyse selon la revendication 5, **caractérisé en ce que** le dialyseur (6") est placé au niveau du dispositif de dialyse de telle sorte que la sortie d'aération (48") est disposée au moins par sections au-dessus de la chambre à sang (38") lorsque le dispositif de dialyse est en position de fonctionnement dans la direction d'accélération gravitationnelle (G).

8. Dispositif de dialyse selon la revendication 6 ou 7, **caractérisé en ce que** le dialyseur (6") est placé au niveau du dispositif de dialyse de telle sorte qu'un liquide dirigé à travers la chambre à sang (38") s'écoule dans une direction diamétralement opposée à l'accélération gravitationnelle (G).

9. Dispositif de dialyse selon la revendication 5, **caractérisé en ce que** le dialyseur est placé au niveau du dispositif de dialyse de telle sorte qu'un liquide dirigé à travers la chambre à sang s'écoule perpendiculairement à l'accélération gravitationnelle (G).
